(19) Europäisches Patentamt
European Patent Office
Office européen des brevets

(11) Publication number: 0 096 130
A1

(12) EUROPEAN PATENT APPLICATION

(21) Application number: 82303854.2

(22) Date of filing: 21.07.82

(51) Int. Cl.³: C 07 D 301/12
C 07 D 303/04

(30) Priority: 11.06.82 US 387341

(43) Date of publication of application: 21.12.83 Bulletin 83/51

(84) Designated Contracting States: BE CH DE FR GB IT LI NL SE

(71) Applicant: Exxon Research and Engineering Company
P.O.Box 390 180 Park Avenue
Florham Park New Jersey 07932(US)

(72) Inventor: Romanelli, Michael Gerald
41 South Portland Avenue
Brooklyn New York(US)

(74) Representative: Dew, Melvyn John et al,
Esso Chemical Ltd. Esso Chemical Research Centre P.O. Box 1
Abingdon Oxfordshire, OX13 6BB(GB)

(54) **Process for the epoxidation of olefins.**

(57) A process for epoxidizing olefins with hydrogen peroxide in the presence of a catalyst composition comprising a Group V element containing Co-catalyst I, such as phenylarsonic acid, and a fluorinated oxygenated Co-catalyst II such as hexafluoroisopropanol is disclosed.

EP 0 096 130 A1

Croydon Printing Company Ltd

Field of the Invention

This invention relates to a process and catalyst composition for the epoxidation of olefins by hydrogen peroxide.

Background of the Invention

The epoxides constitute a class of compounds of which the industrial importance is measured by the tonnages produced and by the diversity of their applications in the field of urethanes, glycols, surface-active agents, plasticizers and numerous other derivatives.

While many specific methods for epoxidizing olefins are known, the most prominent of these methods can generally be divided into four basic categories. For example, the oldest industrial technique for the epoxidation of double bonds is the process known as the chlorohydrin process. In the chlorohydrin process an olefin is reacted with chlorine in an alkaline medium. The yields (based on the chlorine) are unsatisfactory. This process also gives rise to the simultaneous formation of considerable quantities of chlorinated by-products, both inorganic and organic, which products are unsuitable for any known purpose. The disposal of these by-products involves problems of such magnitude that this process may eventually be abandoned.

The second epoxidation method category is generally limited to the epoxidation of ethylene. In accordance with this process ethylene is epoxidized with good yields in the vapor phase by means of molecular oxygen over a catalyst on a silver base. However, this technique is not very useful for the higher carbon olefins because of its lack of selectivity therefor.

A third and more recent category of epoxidation processes is characterized by the use of organic hydroperoxides. In accordance with these processes an olefin is epoxidized catalytically in an organic medium containing an organic hydroperoxide oxidant. In addition to employing a relatively expensive organic hydroperoxide as an oxidant, it is also a characteristic disadvantage of these processes

that the epoxide formation is accompanied by the formation in an equivalent or even greater amount of an alcohol derived from the organic hydroperoxide employed in the process. Consequently, the commercial viability of these processes is always influenced by the ability to economically dispose of the alcohol by-product in addition to the epoxide.

Accordingly, new methods of access to the olefin oxides have been sought which are more direct, more selective and especially are free from the problem of by-products.

This has led to the development of the fourth category of epoxidation processes which are characterized by the use of hydrogen peroxide as the oxidant.

Hydrogen peroxide is in principle a desirable reagent, for the very reason of its oxidizing non-polluting nature. However its reactivity towards olefins is weak or non-existent in the absence of an activating agent which enables a more active per-compound to be formed in-situ. Different processes of epoxidation have therefore been proposed using, for example, organic per-acids such as performic, peracetic or perpropionic acids (see, for example, Belgium Patent No. 535,068). Nevertheless, because of the instability of the epoxides in acid medium, such processes are particularly difficult to put into practice.

It has been proposed to use oxides, oxyacids, or peroxyacids derived from metals such as arsenic, antimony, bismuth, and tungsten (see for example U.S. Patent No. 3,993,673; European Patent Application Publication Nos. 0 008 496 and 0 009 262; and British Patent Specification No. 754,359). However, when such metal catalysts are employed in conjunction with aqueous hydrogen peroxide, the hydrogen peroxide is either rapidly decomposed or the rate of epoxidation is uneconomical. Thus, in an aqueous medium the addition of a metallic catalyst can be self-defeating. Consequently, for optimum performance when

using these catalysts an important requirement of the system is that the hydrogen peroxide be anhydrous. However economic mass production of hydrogen peroxide has become possible owing to developments in the oxidation of secondary alcohols or quinone compounds. These routes to hydrogen peroxide synthesis as practiced commercially ultimately produce dilute aqueous solutions of hydrogen peroxide. Consequently, the cheapest commercially available hydrogen peroxide is generally sold as a 35-40% by weight, aqueous solution thereof. If one has to remove the water from these solutions for use in anhydrous systems, the effective cost of the hydrogen peroxide is increased substantially, thereby increasing the cost of any system requiring the use of anhydrous hydrogen peroxide. It would therefore be economically beneficial to develop a catalytic system which can operate in a medium containing sufficient water such that commercially available aqueous solutions of hydrogen peroxide could be used directly without concentration and/or purification.

U.S. Patent No. 3,778,451 discloses the epoxidation of olefins in an organic solvent medium containing hydrogen peroxide, transition metal compounds, i.e., those of molybdenum, tungsten, vanadium, niobium, tantalum, uranium or rhenium, and a nitrogenous organic base. The organic solvent employed includes alcohols, glycols, esters, linear or cyclic ethers, and certain weak carboxylic acids. However the hydrogen peroxide is employed in substantially anhydrous and concentrated form e.g. contains less than 10%, preferably less than 1% water to limit the production of undesirable hydroxylated by-products.

British Patent Specification No. 1,399,639 discloses the use of a fluorinated ketone, e.g., hexafluoroacetone, or hydrate thereof as a catalyst which can be used in excess quantities to function also as a solvent, or hexafluoroisopropanol (HFIP) as the solvent. However,

this patent does not use the fluorinated ketone or HFIP in conjunction with the specific Group V element containing co-catalysts disclosed herein. Moreover, a majority of reaction times disclosed therein range from about 4 to as high as 270 hours, generally between 5 and 18 hours.

Similarly, it has been reported that the reaction product of hexafluoroacetone with concentrated hydrogen peroxide, i.e., 2-hydroperoxy-hexafluoro-2-propanol, in combination with 30% to 90% $H_2O_2$ (latter gives best results) provides for the catalytic epoxidation of alkenes (see R.P. Heggs, JACS, 2484-2486, 1979). Later, the same Journal reported on arsenated polystyrenes as catalysts for the epoxidation of olefins by aqueous hydrogen peroxide (Jacobson et al, JACS 6946-6950, 1979).

U.S. Patent No. 4,024,165 discloses that the olefin epoxidation process with hydrogen peroxide can be carried out in a fluorinated alcoholic solvent in which all the reactants and catalysts are soluble by using as the catalyst composition a soluble transition metal compound (the disclosed transition metals being limited to molybdenum, tungsten, vanadium, niobium, tantalum, uranium, or rhenium) and a soluble nitrogen-containing compound. In this patent the hydrogen peroxide is present as an aqueous solution, usually 50% by weight (see column 3, lines 19-27). However, the reaction times reported in this reference associated with yields of any significance of olefin oxide range from about 5 to about 8 hours. At reaction times below about 5 hours, the yields of olefin oxide drop substantially and in some instances no reaction at all takes place. Moreover, when either the transition metal compound or the nitrogen containing compound is eliminated from the catalyst composition, yields of olefin oxide also drop significantly. (Compare Examples 1 and 2 wherein in Example 2 elimination of the nitrogen containing compound results in the undesirable polymerization of the olefin oxide; compare also Examples 21 and 24 wherein elimination of the transition metal compound in Example 24 drops the

yield from 70 to 35%).

U.S. Patent No. 4,257,948 discloses a process for epoxidizing acyclic, cyclic, or polycyclic olefins using hydrogen peroxide and a hexachloroacetone catalyst. This patent does not disclose the use of any transition metal catalysts or any other catalyst.

U.S. Patent No. 3,993,673 discloses a process for epoxidizing olefins in the presence of an arsenic catalyst essentially free of tungsten, molybdenum, vanadium and chromium, a hydrogen peroxide oxidant, and an inert organic solvent. Suitable organic solvents include ethers, esters, alcohols, glycols, chlorinated solvents including chlorinated hydrocarbons, and chlorinated aromatics (e.g., chlorobenzene, o-dichlorobenzene, chloroform, and 1,1,2,2-tetra chloro ethane). Although the "hydrogen peroxide can be used in aqueous solutions ... it is preferred to use less water than more" (column 3, lines 43-47). Fluorinated solvents or co-catalysts are not disclosed in this reference. None of the solvents employed include the fluorinated co-catalysts of the present invention.

European Patent Application Publication No. 0 008 496 discloses a polymer supported arsenic heterogeneous catalyst and a process for using the same to oxidize ketones, esters, and olefins in the presence of hydrogen peroxide. When dilute aqueous solutions of hydrogen peroxide are employed as the oxidant, a water immiscible solvent must be employed to avoid contact and hydrolysis of the oxidation products with water. In this embodiment, the substrate to be oxidized as well as the oxidation product are dissolved in the water immiscible solvent creating a two phase organic/aqueous system wherein the hydrogen peroxide is present in the aqueous phase. The polymer supported arsenic catalyst, functioning as a phase transfer catalyst, concentrates at the phase boundary whereat the arsono groups in the polymer are converted by contact with the hydrogen peroxide to perarsono, and this group on contacting the compound to be oxidized in the organic phase

oxidizes it with regeneration of the arsono group. Thus, while suitable water immiscible solvents are disclosed as including chlorinated hydrocarbons, such as chloroform, these solvents are employed solely for their water immiscible property and not for any promoting effect on the arsenic catalyst.

Other phase transfer catalytic systems are disclosed in U.S. Patent No. 3,992,432 and British Patent Specification No. 1,324,763.

European Patent Application Publication No. 0 009 262 discloses the in situ production of hydrogen peroxide and use of the resulting hydrogen peroxide directly in arsenic catalyzed epoxidation reactions of olefins. The in situ production of hydrogen peroxide as well as the epoxidation reaction can be conducted in the presence of aliphatic or cycloaliphatic ethers, aliphatic esters, chlorinated alkanes, chlorinated arenes or sulfolane. None of the solvents disclosed include the fluorinated co-catalysts of the present invention.

British Patent Specification No. 1,452,730 discloses a process for epoxidizing olefins using acetic acid as the catalyst in the presence of aqueous hydrogen peroxide and an inert, chlorinated aliphatic hydrocarbon solvent such as chloroform. The solvent limits the hydrolysis of epoxidized products by the aqueous $H_2O_2$ acetic acid solution. Although the exact mechanism by which this is achieved is not disclosed, it is known that the chlorinated hydrocarbon solvents are water immiscible. Consequently, it is believed that these solvents shield, to varying degrees, the epoxidized product from contact with the aqueous phase by solubilizing the epoxidized product therein.

Other patents which disclose acid catalysis include U.S. Patent No. 3,248,404 (discloses aliphatic and aromatic mono carboxylic acids and their halogenated derivatives as catalysts, e.g., acetic acid, chloroacetic acid, and benzoic acid in conjunction with a sequestering

agent having acid complex forming properties); British Patent Specification No. 1,143,433 (discloses a carboxylic acid cation exchange resin as a catalyst); U.S. Patent No. 2,870,171 (discloses the use of a tungsten acid deposited on an inert support as catalyst); and British Patent Specification No. 754,359 (discloses the use of inorganic per acid catalysts such as the peracids of tungsten, vanadium, and molybdenum as well as heteropoly acids such as the heteropolytungstic acids of arsenic, antimony or bismuth).

British Patent Specification No. 1,302,441 is directed to a process for epoxidizing olefins using hydrogen peroxide and a two component catalyst composition comprising as a first component an organo tin compound having at least one hydroxyl group or coordination group which can be converted to a hydroxyl group in the presence of water or hydrogen peroxide, and as a second component at least one compound containing at least one of molybdenum, tungsten, vanadium, selenium, and boron. Suitable solvents for the reaction include alcohols, e.g., straight chain alcohols, polyhydric alcohols, and cyclic alcohols, as well as epoxides, ketones, and furfurals. Halogenated solvents are not disclosed. While this patent does not require the use of anhydrous or substantially anhydrous hydrogen peroxide, it will be noted that when aqueous solutions of hydrogen peroxide are employed, the reaction times vary from 4 to 24 hours. For example at 90% concentrations of $H_2O_2$ in water (Examples 1, 2 and 4-14) the average reaction time is about 13 hours. However at 70% $H_2O_2$ in water (Examples 15-18) the reaction time is always 20 hours. In contrast when no water is employed with the $H_2O_2$ (Examples 35-52) reaction times are measured in minutes (e.g. 60 to 360 minutes).

Thus, the activity of the catalyst composition of this patent is reduced substantially even at relatively high $H_2O_2$ concentrations in water.

Further, it was reported in the Chemical and Engineering News issue of December 11, 1978 on page 24 that

both Produits Chimiques Ugine Kuhlmann and Union Carbide have each directly oxidized propylene oxide with hydrogen peroxide using an arsenic catalyst. In the former process, hydrogen peroxide of a 70% concentration is employed and for the latter 90% concentration (the author notes that the latter catalyst is adversely affected by the presence of water), with no mention of whether any additional inert diluent or solvent is present.

In summary, substantially all of the disclosures on the epoxidations of olefins to olefin epoxides, particularly propylene to propylene oxide, in which aqueous hydrogen peroxide is used directly in contact with the olefin in either the presence or absence of transition metal catalysts, have eluded commercial development due to one or more economic disadvantages. For example, the aqueous hydrogen peroxide used typically must be substantially above 30% in concentration, and/or the selectivity to propylene oxide is low, or the amount of time required for the reaction is too long. For these reasons, a practical route for direct epoxidation of olefins by aqueous hydrogen peroxide is a long-standing goal in oxidation chemistry. More specifically, it would be extremely economically advantageous to provide a process for epoxidizing olefins using extremely short reaction times while simultaneously achieving comparable or better yields obtainable with prior art techniques particularly, in a dilute aqueous system of $H_2O_2$. Extremely short reaction times enable one to employ simpler plant designs by drastically reducing the size of the reaction vessel since the holding time in the reaction vessel is significantly reduced. Extremely short reaction times also permit one to employ simplified product separation techniques, such as conventional product flash-off procedures, wherein product is continually vaporized directly from the reaction medium, recovered and isolated. If the reaction time is too long, the amount of product vaporized at any given time would be too small to make this technique economically feasible.

The ability to use dilute aqueous solutions of $H_2O_2$ would further increase the flexibility and improve the economics of the process.

Accordingly, there has been a continuing search for processes and catalyst compositions that permit the use of dilute aqueous $H_2O_2$ containing reaction mixtures, where it is advantageous to do so, and which substantially reduce the olefin epoxidation reaction time without sacrificing olefin oxide yield to the point where the process becomes uneconomical. The present invention was developed in response to this search.

Summary of the Invention

In one aspect of the present invention there is provided a process for reacting at least one olefinic compound having at least one ethylenic unsaturation with $H_2O_2$, in the presence of a catalyst composition in a manner and under conditions sufficient to oxidize at least one of said ethylenically unsaturated groups to its corresponding epoxide group. The catalyst composition capable of catalyzing the epoxidation of olefins with $H_2O_2$ comprises at least one Co-catalyst I and at least one Co-catalyst II. The composition of each Co-catalyst is described hereinafter in detail.

Description of the Preferred Embodiments

The present invention is directed to catalyst compositions and processes for using the same to epoxidize olefins with hydrogen peroxide.

More specifically, the catalyst composition comprises at least one Group V element or Group V element-containing compound collectively referred to herein as Co-catalyst I and at least one fluorinated oxygenated compound referred to herein as Co-catalyst II.

The Group V element of Co-catalyst I is selected from the group consisting of As, P, Sb, and Bi. Co-catalyst I comprises inorganic and organic derivative compounds of the Group V elements as well as the elements themselves.

Co-catalyst I is believed to exert its catalytic

effect by reacting with hydrogen peroxide in-situ to form a catalytic intermediate species having, under reaction conditions, an oxidation potential greater than the oxidation potential of hydrogen peroxide alone. The term oxidation potential as used herein is defined to be the potential of a substance to oxidize the ethylenic unsaturation of the olefinic compound to its corresponding epoxide. Thus, the aforesaid intermediate species, such as a peroxy acid, possesses an increased affinity for the electrons of the ethylenic unsaturation relative to the use of hydrogen peroxide in the absence of Co-catalyst I, thereby rendering it more efficient at adding the oxygen from $H_2O_2$ derived moiety of the intermediate across the double bond of the olefin. Co-catalyst II, in some undetermined manner, is believed to facilitate the formation of this intermediate species even in the presence of water, by activating Co-Catalyst I. Consequently, the particular identity of the Co-Catalyst I compound selected for use in the present invention is dictated and controlled by its possession of the capability of interacting with hydrogen peroxide to form said intermediate species under reaction conditions. Typical of such compounds which possess this capability are those which possess an oxy acid functionality or those which can form an oxy acid functionality in-situ. An oxy acid functionality possesses at least one oxo (i.e.O=) group and at least one hydroxy group attached to the aforedescribed Group V element such as $>\overset{O}{\overset{\|}{M}}-OH$ wherein M represents a Group V metal as described herein. In short, Co-catalyst I appears to catalyze the oxidation of the olefin by hydrogen peroxide through the formation of an intermediate, and Co-catalyst II appears to catalyze the formation of the intermediate.

The following is a representative description of Co-catalyst I compounds which are believed to be capable of forming said intermediate with hydrogen peroxide although this description is not intended to be exhaustive.

Accordingly, inorganic Co-catalyst I Group V

element containing compounds include inorganic: oxides, acids such as oxy acids, oxy acid salts, halides, oxy halides, thio halides, sulfides, oxy sulfides and metalides.

Representative inorganic oxides include $As_2O_3$, $As_2O_5$, $P_4O_6$, $P_4O_8$, $P_4O_9$, $P_4O_{10}$, $Sb_4O_6$, $Sb_2O_4$, $Sb_4O_6$, $SbO_2$, $Bi_2O_3$, $5Bi_2O_3 \cdot 3UO_3$, $2As_2O_3 \cdot 12H_2O$ and mixtures thereof.

Representative inorganic oxy acids include $H_2[HPO_3]$, $H_3PO_4$, $H_4P_2O_7$, $H_3PO_2$, $As(OH)_3$, $H_3AsO_4$, $Bi(OH)_3$, and $Sb_2O_3 \cdot (H_2O)_n$.

Representative oxy acid salts include the alkali metal (e.g., Li, Na, K, Rb, Cs), alkaline earth metal (e.g., Be, Mg, Ca, Sr, Ba), ammonium and tetrahydrocarbyl ammonium, preferably tetra lower alkyl (e.g., $C_1$ to $C_{10}$ alkyl) salts of the aforenoted oxy acids, including $NaH_2PO_4$, $Na_2HPO_4$, $KH_2AsO_4$, $K[Sb(OH)_6]$, $NaBiO_3$, $Na_3AsO_4$, tetra ethyl ammonium dihydrogen phosphate, tetra methyl ammonium dihydrogen arsenate, ammonium dihydrogen arsenate and mixtures thereof.

Representative halides and oxy halides include those represented by the structural formulae $PX_3$, $P_2X_4$, $X_3PO$, $X_3PS$, $X_2(O)-P-O-P-(O)-X_2$, $P(H)_a(X)_b$ wherein X is at least one halide independently selected from the group consisting of Cl, F, Br, I, and a+b is 5, including such compounds as for example $P_2Cl_4$, $P_2I_4$, $PHF_4$, $PH_2F_3$, $PH_4Cl$, $PCl_3$, $PCl_5$, $Cl_3PS$, $PF_5$; those represented by the structural formulae $MX_3$ and $MX_5$ where M is As, Sb or Bi, and X is at least one halide as defined above; including such compounds as for example: $AsF_3$, $BiCl_3$, $SbF_3$, $SbCl_3$, $AsF_5$, $SbF_5$, $SbCl_5$, $SbCl_3F_2$, $SbCl_2F_3$, $SbCl_4F$; those represented by the structural formula MOX wherein M and X are defined above including such compounds as for example: SbOCl, BiOCL, and AsOCl; $KSb_2F_7$, and $As_2I_4$.

Representative thio halides include: SbSCl, AsSCl, BiSF, $AsSCl_3$ and mixtures thereof.

Representative sulfides and oxy sulfides include: $As_2S_3$, $As_2S_5$, $BiOS_2$, $Sb_2OS_2$ and mixtures thereof.

Representative examples of suitable metalides include: $As_2Zn_3$.

The most preferred inorganic Co-catalyst I compounds are those containing As as the Group V element, most preferably the As oxides.

Organic Co-catalyst I compounds include those represented by the structural formulae: $R_1ZX'Y$, $R_1R_2ZX'$, and $R_1R_2R_3Z$, wherein: Z is a Group V element in the plus 3 oxidation state selected from the group consisting of P, As, Sb, and Bi; $R_1$, $R_2$ and $R_3$ which may be the same or different are selected from the group of hydrocarbyl radicals consisting of: alkyl, typically alkyl of from about 1 to about 20, preferably from about 1 to about 10, and most preferably from about 1 to about 5 carbons; aryl, typically aryl of from about 6 to about 14, preferably from about 6 to about 10, and most preferably 6 carbons; alkoxy, aryloxy, alkoxyaryl, aryloxyalkyl, aralkyl, alkylthio, arylthio, and alkaryl, wherein the alkyl and aryl groups thereof are as described immediately above in connection with alkyl and aryl respectively; cycloalkyl, typically cycloalkyl of from about 4 to about 20, preferably from about 5 to about 15, and most preferably from about 6 to about 10 carbons; or any two of said $R_1$, $R_2$, and $R_3$ groups together can constitute a cyclic hydrocarbon group having a carbon number as described for cycloalkyl immediately above; substituted: alkyl, aryl, cycloalkyl, alkoxy, aryloxy, alkoxyaryl, aryloxyalkyl, alkylthio, arylthio, alkaryl, and aralkyl, wherein said substituents are selected from the group consisting of: halogen (i.e., Cl, F, Br, I, most preferably F), nitro, and mixtures thereof; and X' and Y which may be the same or different are selected from the group consisting of: hydrogen; halogen (i.e., Cl, F, Br, I), hydroxyl, alkyl, aryl, alkaryl, and aralkyl, said groups being defined as above respectively in connection with $R_1$ to $R_3$; alkoxy wherein the alkyl group thereof is as defined above in connection with $R_1$ to $R_3$; acyloxy $(-O-\overset{\overset{O}{\|}}{C}-R_4)$, mercapto (-SH), alkylthio $(-SR_5)$, and thioacyloxy $(-O-\overset{\overset{S}{\|}}{C}R_6)$, wherein $R_4$, $R_5$, and $R_6$ are alkyl as defined in connection

with $R_1$ to $R_3$.

Representative examples of suitable compounds falling within the scope of the above structural formulae are provided below in chart form wherein each of the variable groups are associated in specific compounds. In the following exemplification, the inclusion of a substituent in parenthesis indicates it can be substituted on any position in the hydrocarbyl group to which it is attached.

FORMULA: $R_1ZX'Y$

| $R_1$ | Z | X' | Y |
|---|---|---|---|
| $C_5H_{11}-$ | As | -H | -H |
| $C_6H_5-$ | As | -Cl | -Cl |
| $C_5H_{11}O-$ | As | -F | -F |
| $C_6H_5O-$ | P | -Br | -Br |
| $CH_3-\emptyset-$ | P | -I | -I |
| $\emptyset-CH_2-O-$ | P | -OH | -OH |
| $(Cl)C_5H_{10}-$ | Sb | $-C_5H_{11}$ | $-C_5H_{11}$ |
| $(F)C_5H_{10}-$ | Sb | $-C_6H_5$ | $-C_6H_5$ |
| $(Br)C_5H_{10}-$ | Sb | $CH_3-\emptyset-$ | $CH_3-\emptyset-$ |
| $(I)C_5H_{10}-$ | Bi | $C_5H_{11}O-$ | $C_5H_{11}O-$ |
| $(NO_2)C_5H_{10}-$ | Bi | $-O\overset{O}{\overset{\|}{C}}-C_5H_{11}$ | $-O\overset{O}{\overset{\|}{C}}-C_3H_7$ |
| $(C_3H_7O)C_5H_{10}-$ | Bi | -SH | -SH |
| $(C_3H_7S)C_5H_{10}-$ | As | $-SC_3H_7$ | $-SC_3H_7$ |
| $C_6H_5S-$ | P | $-O\overset{S}{\overset{\|}{C}}C_3H_7$ | $-O\overset{O}{\overset{\|}{S}}-C_3H_7$ |

---

$\emptyset$ = Phenyl

FORMULA: $R_1R_2ZX'$

| $R_1$ | $R_2$ | Z | X' |
|---|---|---|---|
| $C_5H_{11}-$ | $C_6H_5-$ | As | -H |
| $C_6H_5-$ | $C_5H_{11}O-$ | P | -Cl |
| $C_5H_{11}O-$ | $C_6H_5O-$ | Bi | -F |
| $C_6H_5O-$ | $C_6H_5O-$ | Sb | -Br |
| $CH_3-Ø-$ | $CH_3-Ø-$ | As | -I |
| $Ø-CH_2O-$ | $Ø-CH_2O-$ | P | -OH |
| $CH_3-Ø-O-$ | $CH_3-Ø-O-$ | As | -OH |
| $(Cl)C_5H_{10}-$ | $(Cl)C_5H_{10}-$ | Bi | $-C_5H_{11}$ |
| $(F)C_5H_{10}-$ | $(F)C_5H_{10}-$ | Bi | $-C_6H_5$ |
| $(Br)C_5H_{10}-$ | $(Br)C_5H_{10}-$ | Sb | $-Ø-CH_3$ |
| $(I)C_5H_{10}-$ | $(I)C_5H_{10}-$ | As | $C_5H_{11}O-$ |
| $(NO_2)C_5H_{10}-$ | $(NO_2)C_5H_{10}-$ | As | $-O\overset{O}{\overset{\|}{C}}-C_5H_{11}$ |
| $(C_3H_7O)C_5H_{10}-$ | $(C_3H_7O)C_5H_{10}-$ | P | -SH |
| $(C_3H_7S)C_5H_{10}-$ | $(C_3H_7O)C_5H_{10}-$ | P | $-SC_3H_7$ |
| $C_6H_5S-$ | $C_6H_5S-$ | Bi | $-O\overset{S}{\overset{\|}{C}}-C_3H_7$ |
| 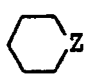 * | * | As | -OH |

*$R_1$ and $R_2$ together constitute a cyclic hydrocarbon

FORMULA: $R_1R_2R_3Z$

| $R_1$ | $R_2$ | $R_3$ | Z |
|---|---|---|---|
| $C_5H_{11}-$ | $CH_3-$ | $CH_3-$ | As |
| $C_6H_5-$ | $C_6H_5-$ | $C_5H_{11}-$ | P |
| $C_5H_{11}O-$ | $C_5H_{11}O-$ | $C_5H_{11}O-$ | Bi |
| $C_6H_5O-$ | $C_6H_5O-$ | $C_5H_{11}O-$ | Sb |
| $CH_3-Ø-$ | $CH_3-Ø-$ | $C_5H_{11}-$ | Bi |
| $Ø-CH_2-$ | $Ø-CH_2-$ | $Ø-CH_2-$ | P |
| $(Cl)C_5H_{10}-$ | $(Cl)C_5H_{10}-$ | $CH_3-$ | As |
| $(F)C_5H_{10}-$ | $CH_3-$ | $CH_3-$ | P |
| $(Br)C_5H_{10}$ | $CH_3-$ | $CH_3-$ | Bi |
| $(I)C_5H_{10}-$ | $C_6H_5O-$ | $CH_3-$ | Sb |
| $(NO_2)C_5H_{10}-$ | $CH_3-$ | $CH_3-$ | Bi |
| $(C_3H_7O)C_5H_{10}-$ | $C_2H_5-$ | $C_2H_5O-$ | P |
| $(C_3H_7S)C_5H_{10}-$ | $CH_3-$ | $CH_3-$ | As |
| $C_6H_5S-$ | $C_2H_5-$ | $C_2H_5-$ | P |

Another class of Group V element containing organic compounds are those in which the Group V element, represented by $Z_1$ hereinbelow, is in the plus 5 oxidation state. Such compounds include those represented by the structural formulae $R_1Z_1(O)X'Y$, $R_1R_2Z_1(O)X'$, and $R_1R_2R_3Z_1(O)$ wherein $R_1$, $R_2$, $R_3$, X' and Y are as defined above in connection with the aforenoted structural formulae wherein the Group V element is in the plus 3 oxidation state.

The preferred Group V element containing organic Co-catalyst I compounds are those of the structural formulae $R_1Z_1(O)X'Y$ wherein X' and Y are hydroxyl and $R_1$ is a halogen substituted, preferably fluorine substituted or unsubstituted hydrocarbyl group selected from alkyl, aryl, aralkyl, alkoxyaryl, most preferably aryl, said hydrocarbyl groups being as defined in connection with $R_1$ above; and $R_1R_2Z_1(O)X'$ wherein $R_2$ is as described in connection with $R_1$ immediately above, and $R_1$, $Z_1$, and X' also are as described immediately above.

Also included within the scope of Co-catalyst I are polymers wherein the Group V element is located in a group pendant to the polymer backbone. A representative example of such a polymeric Co-catalyst I is illustrated by the structural formula:

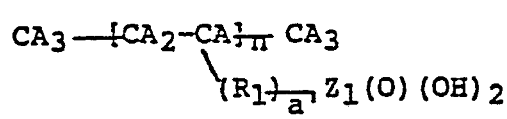

wherein $Z_1$ is as described above, A is independently selected from hydrogen and halogen, preferably fluorine, $R_1$ is a hydrocarbyl group selected from alkyl and aryl, said hydrocarbyl groups being as defined in connection with $R_1$ above, (a') is a number of 0 or 1, and n is a number which can vary from about 5 to 1000, preferably 5 to 500.

Representative compounds falling within the scope of the above formulae for Co-catalyst I are described below wherein each of the variable groups are associated in specific compounds.

FORMULA: $R_1Z_1(O)X'Y$

| $R_1$ | $Z_1$- | X' | Y |
|---|---|---|---|
| $C_5H_{11}-$ | As | -H | -OH |
| $C_6H_5-$ | As | -Cl | -OH |
| $C_5H_{11}O-$ | As | -F | -H |
| $C_6H_5O-$ | P | -Br | -Br |
| $CH_3-\emptyset-O-$ | P | -I | -OH |
| $CH_3-\emptyset-$ | P | -OH | -OH |
| $(Cl)C_5H_{10}-$ | Bi | $-C_5H_{11}$ | $-C_5H_{11}$ |
| $(F)C_5H_{10}-$ | Bi | $-C_6H_5$ | $-C_5H_{11}$ |
| $(Br)C_5H_{10}-$ | Bi | $\emptyset-CH_2-$ | $-C_2H_5$ |
| $(I)C_5H_{10}-$ | Sb | $C_5H_{11}O-$ | $C_5H_{11}O-$ |
| $(NO_2)C_5H_{10}-$ | Sb | $-O\overset{O}{\overset{\parallel}{C}}-C_5H_{11}$ | $-O\overset{O}{\overset{\parallel}{C}}-C_5H_{11}$ |
| $(C_3H_7O)C_5H_{10}-$ | Sb | -SH | -SH |
| $(C_3H_7S)C_5H_{10}-$ | As | $-SC_3H_7$ | $-SC_3H_7$ |
| $C_6H_5S-$ | As | $-O\overset{S}{\overset{\parallel}{C}}-C_3H_7$ | $-O\overset{S}{\overset{\parallel}{C}}-C_3H_7$ |
| $CH_3-CH_2-\emptyset-$ | As | -OH | -OH |
| $CF_3-CF_2-\emptyset-$ | As | -OH | -OH |
| $CH_3-CH_2-C_6H_3F-$ | As | -OH | -OH |
| $CF_3-CF_2-C_6H_2F_2-$ | As | -OH | -OH |
| $C_6H_4F-$ | As | -OH | -OH |
| $C_6H_3F_2-$ | As | -OH | -OH |
| $(NO_2)-C_6H_4-$ | As | -OH | -OH |
| $C_6H_3Cl_2-$ | As | -OH | -OH |
| $CH_3O-C_6H_4-$ | As | -OH | -OH |
| $CH_3-(CH_2)_2$ | As | -OH | -OH |
| $CF_3-(CF_2)_2$ | As | -OH | -OH |
| $\emptyset-(CH_2)_2$ | As | -OH | -OH |
| $C_6H_3F_2-$ | As | -OH | -OH |
| $C_6H_4F-$ | As | -OH | -OH |
| $C_6H_5-$ | As | -OH | -OH |
| $C_3H_7-$ | As | -OH | -OH |

FORMULA: $R_1R_2Z_1(O)X'$

| $R_1$ | $R_2$ | $Z_1$ | $X'$ |
|---|---|---|---|
| $C_5H_{11}-$ | $C_5H_{11}-$ | As | $-OH$ |
| $C_6H_5-$ | $C_6H_5-$ | As | $-OH$ |
| $C_5H_{11}O-$ | $C_5H_{11}O-$ | As | $-Br$ |
| $C_3H_7-$ | $C_3H_7O-$ | P | $-H$ |
| $CH_3-\emptyset-$ | $CH_3-\emptyset-$ | P | $-OH$ |
| $(Cl)C_5H_{10}-$ | $(Cl)C_5H_{10}-$ | P | $-OH$ |
| $C_5Cl_{11}-$ | $C_5Cl_{11}-$ | Bi | $C_5H_{11}-$ |
| $C_5F_{11}-$ | $C_5F_{11}-$ | Bi | $C_5H_{11}-$ |
| $(NO_2)C_5H_{10}-$ | $(NO_2)C_5H_{10}-$ | Bi | $-C_2H_5$ |
| $C_6H_4F-$ | $C_6H_4F-$ | Sb | $C_5H_{11}O-$ |
| $C_5H_{11}\overset{O}{\overset{\|}{C}}-O-$ | $C_5H_{11}\overset{O}{\overset{\|}{C}}-O-$ | As | $-O\overset{O}{\overset{\|}{C}}-C_5H_{11}$ |
| $C_3H_7-$ | $C_3H_7-$ | Sb | $-SH$ |
| $C_6H_5-$ | $C_6H_5-$ | As | $-SC_3H_7$ |
| $C_3H_7-$ | $C_6H_5-$ | Bi | $-SC_3H_7$ |
| $CH_3-$ | $CH_3-$ | As | $-OH$ |
| $CH_3-CH_2-\emptyset-$ | $CH_3-CH_2-\emptyset-$ | As | $-OH$ |
| $CH_3-CH_2-C_6H_3F-$ | $CH_3-CH_2-C_6H_3F-$ | As | $-OH$ |
| $CF_3-CF_2-C_6H_2F_2-$ | $CF_3-CF_2-C_6H_2F_2-$ | As | $-OH$ |
| $C_6H_4F-$ | $C_6H_4F-$ | As | $-OH$ |
| $C_6H_3F_2-$ | $C_6H_3F-$ | As | $-OH$ |
| $(NO_2)-C_6H_4-$ | $(NO_2)-C_6H_4-$ | As | $-OH$ |
| $C_6H_3Cl-$ | $C_6H_3Cl-$ | As | $-OH$ |
| $CH_3-O-C_6H_4$ | $CH_3-O-C_6H_4-$ | As | $-OH$ |

FORMULA: $R_1R_2R_3Z_1(O)$

| $R_1$ | $R_2$ | $R_3$ | $Z_1$ |
|---|---|---|---|
| $C_5H_{11}-$ | $C_5H_{11}-$ | $C_5H_{11}-$ | As |
| $C_6H_5-$ | $C_6H_5-$ | $C_6H_5-$ | As |
| $C_6H_5O-$ | $C_6H_5O-$ | $C_6H_5O-$ | As |
| $C_6H_5-$ | $CH_3-$ | $CH_3-$ | Sb |
| $C_6H_4F-$ | $C_6H_4F-$ | $C_6H_4F-$ | Bi |
| $NO_2C_5H_{10}-$ | $C_5H_{11}-$ | $C_6H_5-$ | P |
| $CH_3CH_2-$ | $CH_3CH_2-$ | $CH_3CH_2-$ | As |

FORMULA: $CA_3-[CA_2CA]_n-CA_3$

$\qquad\qquad(R_1)_{a'}-Z_1(O)(OH)_2$

| A | $R_1$ | a' | $Z_1$ |
|---|---|---|---|
| H- | N/A | 0 | As |
| H- | $-C_6H_4-$ | 1 | As |
| H- | $-CH_2-$ | 1 | As |
| F- | N/A | 0 | As |
| F- | $-C_6H_4-$ | 1 | As |
| F- | $-CH_2-$ | 1 | As |
| H- | $-C_6H_4-$ | 1 | P |
| F- | $-C_6H_4-$ | 1 | P |
| H- | $-CH_2-$ | 1 | Bi |
| F- | $-CH_2-$ | 1 | Bi |
| H- | $-C_6H_4-$ | 1 | Sb |
| F- | $-CH_2-$ | 1 | Sb |

Also included as suitable Co-catalyst I are those having at least two of the aforedescribed Group V elements such as 1-diphenyl-phosphino-2-diphenyl arsinoethane and 2-arsenato-ethyl-triphenyl phosphonium bromide.

The preferred class of organic Group V element containing compounds include the arsenic containing compounds. Of this class, the most preferred organic compounds include: arsenictriethoxide, phenylarsonicacid, diphenylarsinic acid, dimethylarsinic acid, 2-nitro-4-methylphenylarsonic acid, 4-methylphenylarsinic acid, n-propyl arsonic acid, 4-hydroxy-3-nitrophenylarsonic acid and mixtures thereof.

The fluorinated organic compound of Co-catalyst II contains: at least one oxygenated functional group selected from the group consisting of hydroxy, ester, and ether, said oxygenated functional group being attached to a saturated aliphatic carbon; and at least one fluorine group. Thus, Co-catalyst II comprises fluorinated alcohols, ethers, esters, and thioesters, fluorinated compounds of mixed oxygenated functional groups, fluorinated polymers containing pendant oxygenated functional groups, and mixtures of the aforenoted fluorinated organic compounds.

A majority of these compounds may be represented by the structural formula:

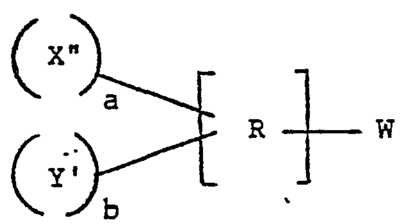

(I)

wherein: W is an oxygenated functional group selected from the group consisting of -OH, $-SO_3R_7$, $-COOR_8$, $-C-O-R_9$, $R_7$, $R_8$, $R_9$, being independently selected from the group consisting of halogen substituted (preferably F substituted) and unsubstituted alkyl of from about 1 to about 10, preferably from about 1 to about 5, most preferably from about 1 to about 3 carbons; R is a hydrocarbyl group selected from

the group consisting of: alkyl of from about 1 to about 10, preferably from about 1 to about 8 (e.g., 2 to 8) carbons, most preferably from about 2 to about 5 carbons; aralkyl wherein the aryl portion thereof has from about 6 to about 10, preferably 6 carbons and the alkyl portion which is as defined immediately above; and cycloalkyl of from about 4 to about 12, preferably from about 5 to about 10, most preferably from about 5 to about 8 carbons. Also, in structural formula I, each X" is independently selected from the group consisting of: alkyl as described above in connection with R, hydrogen, halogen (i.e., F, Cl, Br, I), preferably fluorine; Y' is attached to the aliphatic portion of said R hydrocarbyl group and is independently selected from the group consisting of hydrogen, halogen, -OH, $-SO_3R_{11}$, $-COOR_{12}$, and $-C-O-R_{13}$, $R_{11}$, $R_{12}$, and $R_{13}$ being as defined in connection with $R_7$, $R_8$, and $R_9$ respectively provided that when Y' is hydroxy, its position of substitution on the R group is such that no aliphatic carbon of R contains more than one hydroxy group; the letter (a) is a number of at least 1; at least one X" is fluorine, and a + b is a number equal to the total number of available carbon bonding sites on the unsubstituted hydrocarbyl R group. For the purposes of the present invention, the total number of available carbon bonding sites on an unsubstituted hydrocarbyl group is defined herein to be the total number of replaceable hydrogens on said unsubstituted hydrocarbyl group.

More preferably in structural formula I, X" is independently selected from the group consisting of hydrogen and halogen, e.g., fluorine, Y' is hydroxyl; and W is -OH.

Preferably, at least one fluorine constituting X" is located on a carbon which is between about 2 and about 5, preferably between about 2 and about 4, most preferably between about 2 and 3 carbons

removed from the oxygen present in the oxygenated functional group constituting W. The number of carbons removed from the oxygen of the oxygenated functional group is determined herein by counting outward from the oxygen of the oxygenated functional group, the number of carbons between said oxygen and the fluorine substituent. Said another way, it is preferred that at least 1, preferably between 2 and about 10, most preferably between about 2 and about 6 fluorines constituting X" be located in the vicinity of the oxygenated functional group (i.e., sufficiently close that the electron withdrawing effect of the fluorine can be exerted on the oxygen of the oxygenated functional group). For example, where the oxygenated functional group contains a carbon, it is most preferred that at least 1, preferably at least about 2 fluorines constituting X" be located on the carbon atom adjacent to the carbon of the oxygenated functional group (i.e., 2 carbons removed from the oxygen of oxygenated functional group). Where the oxygenated functional group does not contain a carbon, it is most preferred that at least 1, preferably at least 2 fluorines constituting X" be located on the carbon twice removed from the functional group (e.g., $-CF_2-CH_2-OH$) and/or 3 carbons removed from the oxygen of the oxygenated functional group (e.g., $CF_2-CF_2-CH_2OH$, or $CF_2-CH_2-CH_2OH$, respectively).

The number ratio of the oxygenated functional group(s) to fluorine generally will vary from about 2:1 to about 2:20, preferably from about 2:2 to about 2:6, most preferably from about 2:2 to about 2:4.

It is to be understood that in selecting the identity and position of substituents X", Y', and W, that such selection be made to avoid the possibility of any of these functional groups reacting with each other, i.e., X", Y' and W bear an inert relationship. For example, an hydroxyl group and a fluorine group attached to the same aliphatic carbon are reactive in aqueous media, the reaction product being HF. It is for this reason that the

substituents X", Y', and W are inert with respect to each other on the same compound and preferably with respect to the epoxide product.

Individual classes of Co-catalyst II compounds are discussed hereinafter.

One such class includes fluorinated primary, secondary, and tertiary, mono or polyhydroxy aliphatic alcohols, (e.g., about 2 to about 4 hydroxyl groups). The term "alcohol" as defined herein is intended to exclude hydrated ketones which possess the functionality $-C(OH)_2-$. Secondary monohydroxy fluorinated alcohols having from about 2 to about 20, preferably from about 2 to about 10, most preferably from about 2 to about 6 carbons are most preferred.

Such aliphatic alcohols can be represented by the structural formula:

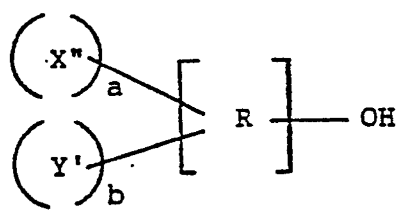

(II)

wherein X", Y', R, a, and b are as defined above in connection with structural formula I in the broad as well as more preferred embodiments. More preferably in structural formula (II) R is selected from the group consisting of $C_2$ to $C_8$ alkyl and a $C_8$ aralkyl; X" is fluorine and Y' is hydrogen.

Typically the number ratio of hydroxyl groups to fluorine groups in the above structural formula is as defined hereinabove.

A still more preferred class of fluorinated aliphatic alcohols can be represented by the structural formula:

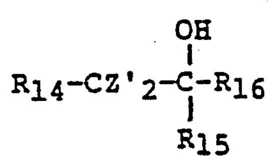

(III)

wherein $R_{14}$, $R_{15}$, and $R_{16}$ which may be the same or different are selected from the group consisting of hydrogen, and a substituted or unsubstituted hydrocarbyl radical group selected from alkyl of from about 1 to about 10, preferably from about 1 to about 5 carbons; aryl of from about 6 to about 10, preferably 6 carbons; aralkyl and alkaryl wherein the alkyl and aryl groups thereof are as defined immediately above, said substituents when present being selected from halogen (preferably fluorine), and hydroxy and being substituted on the aliphatic portion of said $R_{14}$, $R_{15}$, and $R_{16}$ hydrocarbyl groups, with the proviso that when hydroxyl and halogen substituents are present together on any of the aforenoted alkyl groups, such halogen substituents are located on a carbon different from the hydroxylated carbon, preferably from about 2 to about 5, most preferably from about 2 to about 3 carbons removed from the hydroxyl group. Furthermore, any two of the $R_{14}$, $R_{15}$, and $R_{16}$ groups together can constitute a halogen (preferably F) and/or hydroxyl substituted or unsubstituted cycloalkyl group of from about 4 to about 12, preferably from about 5 to about 8 carbons, subject to the same proviso. Each Z' in structural formula III is independently selected from the group consisting of hydrogen, and halogen (preferably F and Cl) with the proviso that at least one Z' group is F.

A still narrower and preferred class of Co-catalyst II compounds can be represented by the structural formulae:

$CF_3-(CF_2)_{a'}CHOHCF_3$ (IV)

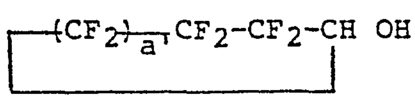

( V)

$CF_3-CHOH-(CF_2CHOH)_{a'}-(CF_2-CF_2)_{b'}-CF_3$ (VI)

wherein (a') and (b') are numbers which can independently vary from about 0 to about 10, preferably from about 1 to about 8, and most preferably from about 1 to about 5.

Typically the number ratio of hydroxyl groups to fluorine groups on the compounds represented by structural formula III is from about 2:1 to about 2:20, preferably from about 2:2 to about 2:6, and most preferably from about 2:2 to about 2:4.

Representative examples of suitable fluorinated alcohols include 3,3,3,2,2-pentafluoro-1-propanol; 2,2,2-trifluroethanol; hexafluoroisopropanol; 3-fluoro-3-chloro-1,1,1-trifluoro-2-butanol; 3,3,4,4-tetrafluoro-2-heptanol; 3,3-difluoro-2,4-pentanediol; 1,1,3,3,4,4,4-heptafluoro-2-methyl-2-butanol; 2,2,3,3,4,4,4-heptafluoro-1-butanol; 1,1,1,3,3,4,4,4-octafluoro-2-butanol; 2-phenyl-2-difluoro-1-ethanol; 1-phenyl-3-difluoro-isopropanol; 3-(2-ethyl-phenyl)-2-fluoro-1-ethanol; (3-phenyl-3-fluoro) -2-decanol; 3-(2-methylphenyl)-3-fluoro-2-pentanol; 1,1,1,3,3,4,4,5,5, 6,6,6-2-dodecanol.

The most preferred fluorinated alcohol is hexafluoroisopropanol.

A further class of suitable Co-catalyst II fluorinated organic compounds is the fluorinated ethers including monoethers and polyethers as from about 1 to about 4 ether functional groups.

Such ethers may be represented by the structural formula:

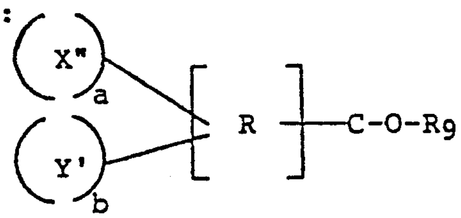

(VII)

wherein X", Y', R, $R_9$, a and b are as described in connection with formula I in the broad, as well as more preferred embodiments. Still more preferably in structural formula (VII), R is selected from the group consisting of $C_1$ to $C_8$ alkyl; X" is fluorine, and Y' is hydrogen.

Typically the number ratio of ether groups to fluorine constituting X" is from about 2:1 to about 2:20 preferably from about 2:2 to about 2:6, most preferably

from about 2:2 to about 2:4.

A more preferred class of fluorinated ethers is represented by the structural formula:

$$R_{23}\text{---}CG_2 \quad \text{-O-} \quad CG_2\text{---}R_{24} \qquad \text{(VIII)}$$

wherein $R_{23}$ and $R_{24}$ which may be the same or different are selected from the group consisting of hydrogen; halogen, preferably F and Cl; and halogen substituted (preferably F substituted) and unsubstituted: alkyl of from about 1 to about 10, preferably 1 to about 5 carbons; and alkoxy wherein the alkyl group is as defined immediately above. Furthermore, $R_{23}$ and $R_{24}$ together can constitute a halogen substituted (preferably F substituted) or unsubstituted cycloalkyl group of from about 4 to about 12 preferably from about 5 to about 8 carbons. G in structural formula (VIII) is independently selected from the group consisting of hydrogen and halogen (preferably Cl, most preferably F) with the proviso that at least one G group is fluorine.

A class of suitable polyether fluorinated compounds can be represented by the structural formula:

$$CG_3 \text{---} \left[ OCG_2\text{-}R_{25} \right]_n \text{---} CG_3 \qquad \text{(IX)}$$

wherein G is as defined in connection with structural formula (VIII); $R_{25}$ is selected from hydrogen; halogen; and a halogen substituted (preferably F substituted) or unsubstituted: alkyl group of from about 1 to about 10, preferably from about 1 to about 5 carbons; and n is a number of from about 1 to about 5, preferably from about 1 to about 3.

Still more preferably in structural formulae (VIII) and (IX), the halogen independently constituting any of G, $R_{23}$, $R_{24}$, $R_{25}$, and halogen substituted $R_{23}$, $R_{24}$, and $R_{25}$ is fluorine.

Representative examples of suitable fluorinated ethers include perfluoro-1-methoxybutane; perfluoro-1-methoxy-2-propoxyethane; perfluorotert butoxy methane and

mixtures thereof.

Suitable esters which can function as Co-catalyst II include those represented by the structural formula I wherein W is $-COOR_8$ or $SO_3R_7$. Preferably R in structural formula I, when W is as described immediately above, is alkyl, X" is fluorine and Y' is hydrogen.

Such esters include those containing one or more, e.g., 2 to about 4, ester functional groups.

Typically the number ratio of ester functional groups to fluorine can vary from about 2:1 to about 2:20, preferably from about 2:2 to about 2:6, most preferably from about 2:2 to about 2:4.

A more limited preferred class of fluorinated esters can be represented by the structural formulae:

$$CF_3-\left(CF_2\right)_{a'}-CO_2-CF_2-CF_3 \qquad (X)$$

$$CF_3-\left(CF_2-CO_2\right)_{a'}-CF_2-CF_3 \qquad (XI)$$

wherein a' is a number as described in connection with structural formula (IV).

Representative examples of suitable fluorinated esters include: perfluoroethyl-2,2,3,3,3-pentafluoropropanoate; perfluoromethyl-2,2,3,3,4,4,4-heptafluorobutanoate; perfluoropropyl-2,2,2-trifluoroethanoate and mixtures thereof.

Fluorinated compounds of mixed oxygenated functional groups include fluorinated: hydroxy esters and hydroxy ethers.

Representative examples of suitable fluorinated mixed oxygenated functional groups containing compounds include methyl-2,2,3,3-tetrafluoro-4-hydroxybutanoate.

Also included within the scope of the present invention are Co-catalyst II fluorinated oxygenated functional group containing polymers wherein the oxygenated functional groups defined by W in structural formula I are located pendant to the polymer backbone and/or are integrated

as part of the polymer backbone.

Such polymers preferably are homopolymers or random co-polymers and preferably are derived from fluorinated vinyl monomers having pendant oxygenated functional groups or from fluorinated monomers which polymerize through the oxygenated functional group.

Co-catalyst II fluorinated polymers wherein the oxygenated functional group is pendant to the polymer backbone include those represented by the structural formula:

$$CF_3 - \left[ CF_2 - \underset{\displaystyle Q}{\underset{|}{C}}R_{26} \right]_n - CF_3 \qquad (XII)$$

wherein Q is an oxygenated functional group selected from the group consisting of $-OH$, $-SO_3R_7$, $-COOR_8$, $-C-O-R_9$, $R_7$, $R_8$, and $R_9$ being as defined in connection with structural formula I, $R_{26}$ is hydrogen or halogen, preferably F, with the proviso that when Q is $-OH$, $R_{26}$ is hydrogen, and n is a number which can vary from about 5 to about 1000, preferably from about 5 to about 500.

Preferred polymers are those wherein Q is -OH.

Fluorinated Co-catalyst II polymers wherein the oxygenated functional group is part of the polymer backbone include those represented by the structural formula:

$$CF_3 - \left[ CF_2 - CF - Q' \right]_n - CF_3 \qquad (XIII)$$

wherein Q' is selected from the group consisting of -O- and $-\overset{O}{\overset{\|}{C}}-O-$, and n is as defined in connection with structural formula XII.

When the polymer is a solid, it is preferred to dissolve it in a suitable inert solvent or alternatively the polymeric Co-catalyst II can operate in a heterogeneous phase if desired.

Co-catalysts I and II are employed to enhance the epoxidation reaction rate of olefins and/or selectivity to epoxide, said epoxidation reaction being achieved using $H_2O_2$ as the oxidant.

Accordingly, olefins which can be epoxidized using $H_2O_2$ and which can be employed in the present invention contain at least one ethylenic unsaturation and are conventional in the art. Typical of such olefins are those represented by the structural formula:

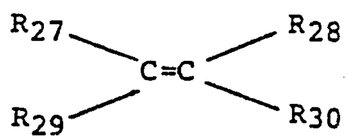

(XIV)

wherein $R_{27}$, $R_{28}$, $R_{29}$ and $R_{30}$, which may be the same or different, are selected from the group consisting of hydrogen; substituted or unsubstituted: alkyl, aryl, alkaryl, and aralkyl hydrocarbyl groups, said hydrocarbyl groups being preferably as defined in connection with R of structural formula I; or any two of said $R_{27-30}$ groups together can constitute a cycloalkyl group typically of from about 4 to about 12, preferably from about 5 to about 8 carbons.

Representative olefins which can be epoxidized and contain at least one ethylenic saturation include: ethylene, propylene, butene-1, butene-2, pentene-1, pentene-2, isobutene, pentene-1, pentene-2, hexene, isohexene, heptene, 3-methylhexene, octene-1, isooctene, nonene, decene, dodecene, tridecene, pentadecene, octadecene, eicosene, docosene, tricosene, tetracosene, pentacosene, butadiene, pentadiene, hexadiene, octadiene, decadiene, tridecadiene, eicosadiene, tetracosadiene, cyclopentene, cyclohexene, cycloheptene, methylcyclohexene, isopropylcyclohexene, butylcyclohexene, octylcyclohexene, dodecyclohexene, acrolein, acrylic acid, methyl methacrylate, styrene, cholesterol, etc. The preferred olefins are propylene, isobutylene, styrene, allyl alcohol and allyl chloride. The most preferred olefin is propylene.

The components in the catalyst composition of the present invention are employed in amounts effective to increase yields and/or selectivities of epoxide relative to the absence of said components, typically within a reaction time of from about 2 minutes to about 3.5 hours, preferably from about 10 minutes to about 2 hours and most preferably from about 15 minutes to about 1 hour.

The process of the present invention is conducted by contacting at least one olefin containing at least one ethylenic unsaturation and $H_2O_2$, preferably in a liquid phase, in the presence of said catalyst composition under conditions and in a manner sufficient to oxidize at least one of said ethylenically unsaturated groups to its corresponding epoxide group.

The $H_2O_2$ can be employed in anhydrous form or as an aqueous solution. Such aqueous solutions typically will contain from about 3 to about 99.9%, preferably from about 20 to about 75%, and most preferably from about 20 to about 45% (e.g., 25 to 35%), by weight, $H_2O_2$ based on the total weight of the aqueous solution. While it is generally recognized in the art that yields of epoxide product are highest if the water content of the reaction mixture is kept to a minimum, it is a particular advantage of the present invention that reaction rate enhancement and yields are not particularly adversely affected when operating at water levels in the aqueous $H_2O_2$ solution of about 60 to 75% (e.g., 70%) by weight thereof including water produced by the reaction.

Consequently, the economics of the overall process are substantially improved due to the ability to use commercially produced aqueous solutions of $H_2O_2$ directly, without the need to concentrate such solutions by removing water therefrom.

While it is generally desirable to employ the reactants (i.e., olefin and $H_2O_2$) in approximately stoichiometric proportions, e.g., one mole of $H_2O_2$ per mole of ethylenic linkage to be epoxidized, an excess of either

reactant is acceptable and in many instances preferable since it actually results in easier process control and a more economic process.

The preferred mode of reacting the olefin and $H_2O_2$ is conducted in a liquid reaction medium and preferably in the presence of a solvent miscible with both reactants. While it is not necessary that the reaction medium exist as a homogeneous phase, it is preferred that it does. Furthermore, it is also contemplated that the $H_2O_2$ can be formed in-situ from the oxidation of quinones in accordance with EPA number 9262.

Because the Co-catalyst II fluorinated organic compound can be used in excess relative to the effective catalytic amount, it can function not only as a co-catalyst but also as the primary solvent for the reaction medium when employed in such excess to achieve a homogeneous liquid reaction phase. However, it may be more economically advantageous to employ less expensive solvents for this purpose.

Suitable alternative less expensive solvents, are organic, and inert in the reaction medium. Such alternative inert organic solvents include benzene, toluene, xylene, pentane, hexane, heptane, non-fluorinated: alcohols, e.g., isopropanol, ketones, e.g., acetone, carboxylic acids, e.g., propanoic acid and esters, e.g., ethyl acetate.

In carrying out a preferred embodiment of the invention, olefin or olefin and $H_2O_2$ are introduced into a liquid reaction mixture comprising Co-catalyst I, Co-catalyst II and optionally inert organic solvent. Preferably the reaction mixture also contains an aqueous solution of the $H_2O_2$ prior to introducing the olefin thereto.

Thus, the initial preferred reaction mixture prior to olefin introduction will typically comprise:
(a) an aqueous $H_2O_2$ solution (as defined above) in an amount of from about 0.5 to 25%, preferably from about 2 to about 20%, and most preferably from about 10 to about 15%, by weight, based on the weight of the reaction medium exclusive of the weight of olefin and Co-catalyst I;

(b) Co-catalyst II in an amount of from about 10 to about 99.5%, preferably from about 30 to about 90%, and most preferably from about 70 to about 90%, by weight, based on the weight of the reaction medium exclusive of the weight of olefin and Co-catalyst I; (c) inert organic solvent in an amount of from about 0 to about 90%, preferably from about 0 to about 50%, and most preferably from about 10 to about 30%, by weight, based on the weight of the reaction medium exclusive of the weight of olefin and Co-catalyst I; and (d) Co-catalyst I in an amount sufficient to achieve from about 0.001 to about 2, preferably from about 0.005 to about 1.0, and most preferably from about 0.01 to about 1.0 g atom of the Group V element in Co-catalyst I per liter of reaction medium inclusive of components (a) through (c) recited above.

If the aqueous solution of $H_2O_2$ is added simultaneously with the olefin, it is added in amounts sufficient to eventually achieve the reaction mixture composition as described above.

The olefin is introduced into, and contacted with, the reaction mixture in an amount and in a manner sufficient to achieve at least an initial molar ratio of olefin to $H_2O_2$ therein of typically from about 0.8:1 to about 50:1, preferably from about 1:1 to about 30:1, and most preferably from about 1:1 to about 10:1. Thus, in most instances it is preferred to maintain an excess of olefin in the reaction mixture.

The reaction temperature can vary widely although it is preferred to maintain the reaction mixture in the liquid phase. Accordingly, typical reaction temperatures will vary from about 20 to about 150°C, preferably from about 40 to about 120°C, and most preferably from about 50 to about 90°C. The reaction pressure is not critical and can be atmospheric, sub-atmospheric or super-atmospheric.

Typically, the reaction pressure is controlled in a manner sufficient to keep the olefin, reactants and

Co-catalyst II in a liquid phase. Furthermore, it is highly desirable to conduct the reaction under the autogeneous pressure generated by the reactants at the temperature selected.

The process may be run in a batch mode or a stepwise mode or a continuous mode where either one or both the olefin or hydrogen peroxide may be added simultaneously or sequentially to maintain reactant concentration as they are consumed.

Additionally, the process may be run in either of the aforementioned modes by altering the reaction conditions, and/or, the reactant, solvent, or catalyst concentrations during the course of the reaction. Thus, the process may be run by changing the temperature, pressure, catalyst concentration, hydrogen peroxide concentration or olefin concentration.

The practice of the process of the present invention within the aforenoted reaction times is capable of achieving epoxide yields as high as 100%.

As used herein, percent epoxide selectivity is defined as:

$$\text{selectivity (\%)} = \frac{\text{moles of epoxide produced}}{\text{moles of } H_2O_2 \text{ converted to organic products}} \times 100$$

To determine selectivity to by-product, the moles of epoxide in the above equation is replaced by moles of by-product.

Conversion is reported in two forms, namely, conversion of $H_2O_2$ to organic products and total conversion of $H_2O_2$ including organic products as well as decomposition products of $H_2O_2$ such as oxygen and water. Total conversion is determined as follows:

$$\text{Total conversion (\%)} = \frac{\text{moles of } H_2O_2 \text{ reacted}}{\text{moles of } H_2O_2 \text{ charged}}$$

Conversion to organics is determined as follows:

$$\text{Conversion to organics (\%)} = \frac{\text{moles of } H_2O_2 \text{ reacted to form organic products}}{\text{moles of } H_2O_2 \text{ charged}}$$

Unless otherwise specified, total conversion is determined by iodometric titration of residual peroxide remaining after reaction and conversion to organics is determined by gas chromotography. Most of the conversions disclosed herein are reported as being determined by two different analytical methods because unpassivated stainless steel reactors are well known to cause decomposition of hydrogen peroxide (see for example, "Hydrogen Peroxide" by Schumb, W., Satterfield, R., and Wentworth, R., American Chemical Society Monograph, published by Reinhold (1955)). This decomposition of hydrogen peroxide can be eliminated or minimized to a negligible degree by passivating the stainless steel using conventional techniques such as treatment with nitric acid. Consequently, for practical purposes the advantages of the present invention are observed from conversion to organic products and selectivity of these organic products to epoxide. Total conversion is reported herein in the interest of completeness but has no real bearing on the performance of the process of the present invention. The yield of epoxide can be calculated as the product of conversion to organics and selectivity.

Because the Co-catalyst composition of the present invention greatly increases the rate of the epoxide forming reaction, it becomes economically feasible to remove the epoxide product as fast as it is formed by, for example, a product flash-off technique. This permits immediate removal of the epoxide product and substantially reduces the chances for undesirable hydrolysis reaction of the epoxide to corresponding glycol to occur. Thus, a volatile epoxide may be removed by vaporization from the reaction medium preferably by use of a stripping gas. Alternatively, volatile and non-volatile epoxides may also be removed by preferential extraction into a separate solvent phase which then in turn may be removed from the reaction medium preferably by decanting.

Further isolation of the resulting epoxide from volatilized constituents of the reaction medium or from the

extraction medium can be accomplished by fractional distillation to yield the substantially pure epoxide in cases where the epoxide is relatively low boiling.

It is to be understood that while the Group V element containing compounds and fluorinated oxygenated compounds are referred to herein generally as co-catalysts, the exact mechanistic relationship by which these classes of compounds exert their reaction time reducing effect is not entirely understood. Consequently, the use of the term "co-catalyst" is meant to include the possibility of a promoter: catalyst type of relationship between each of these respective classes of compounds.

The utilities of the epoxidized products produced in accordance with the process of the present invention are well known and include use as intermediates in the preparation of polyesters, polyurethanes, detergent products, and the like.

The following examples are given as specific illustrations of the claimed invention. It should be understood, however, that the invention is not limited to the specific details set forth in the examples. All parts and percentages in the examples as well as in the remainder of the specification are by weight unless otherwise specified. Unless otherwise specified, all reactions reported herein below are conducted in a manner such that olefin is available for reaction with $H_2O_2$ in an amount in excess of the stoichiometric amount relative to the $H_2O_2$.

EXAMPLE 1

Into a 100 ml. three-neck flask equipped with a reflux condenser, thermometer, and magnetic stirrer are placed 4.9320g of cyclohexene (60.0 mmole), 2.2735g of 30% $H_2O_2$ in water (20.1 mmole), 24.80g of hexafluoroisopropanol (Co-catalyst II), and 0.0806g (0.401 mmole) of phenylarsonic acid (Co-catalyst I). The reaction is stirred and heated at 65°C for 1 hour. Gas Chromatography (i.e. G.C.) analysis of the reaction mixture indicates the formation of 0.437g

(4.5 mmole) of cyclohexene oxide and 0.540g (4.6 mmole) of 1,2-cyclohexandiol. The epoxide selectivity is accordingly 48.9%, and the conversion to organics is 45.4%.

Comparative Example 1

The reaction conditions of Example 1 are repeated using 4.9620g (60.4 mmole) of cyclohexene, 2.291g (20.3 mmole) of 30% $H_2O_2$ in water, 25.85g of hexafluoroisopropanol, 0.1560g (0.7 mmole) of heptafluorobutyric acid and 0.0804g (0.400 mmole) of phenylarsonic acid. A G.C. analysis, of the reaction mixture after 1 hour indicates the formation of 0.121g (1.3 mmole of cyclohexene oxide, and 0.424g (3.7 mmole) of 1,2-cyclohexandiol. The epoxide selectivity is accordingly 25.2%, and the conversion to organics is 24.2%. The presence of heptafluorobutyric acid thus reduces both the selectivity and conversion relative to Example 1.

Furthermore, the results of Example 1 when employing cyclohexene as the olefin compared to the results of examples employing propylene reflect the decreased stability of cyclohexene oxide versus propylene oxide.

EXAMPLE 3:

A 150 ml. stainless steel bomb is charged with 24.9g of 1,1,1,3,3,3-hexafluoroisopropanol, 2.3118g of 30% $H_2O_2$ in water (20.4 mmole), 0.0805g phenylarsonic acid (0.400 mmole) and 1.0258g heptane, as an internal standard. The bomb is heated to 74$^{O}$C in a water bath and pressurized to 145 psig with propylene to dissolve olefin in the reaction medium in excess of stoichiometry. Propylene is continually added in excess to maintain pressure. After 110 min., a G.C. analysis shows the presence of 1.026 (17.7 mmole) of propylene oxide. The epoxide selectivity is 100%, and the conversion to organics is 87.0%.

EXAMPLES 4-9:

In general accordance with the procedures preceding Example 3, and olefin dissolved in excess of stoichiometry several reaction mixtures are prepared and the reactants reacted. The composition of the reaction mixtures

and reaction conditions are summarized at Table I, runs 1 to 12, as are the results thereof. These results illustrate that even at reaction times of about 5 minutes conversions from 20 to 30% are achieved at epoxide selectivities above 90%. After a reaction time of only about 1 hour, conversion is increased substantially. These results are all achieved using dilute aqueous $H_2O_2$ solutions.

EXAMPLE 10:

A 300 ml. stainless steel autoclave is charged with 0.3215g phenylarsonic acid, 103.89g hexafluoroisopropanol, 12.72g of 30% aqueous hydrogen peroxide, and heated to 70°C. Propylene is then added to 140 psig in excess of stoichiometry as needed. After 5 minutes of reaction to organics, there is obtained 0.793g of propylene oxide, at a conversion of 12%, and epoxide selectivity of 100%. After 30 minutes of reaction, the sample shows the production of 2.05g of propylene oxide, at a conversion to organics of 32%, total conversion of 20.9%, and selectivity of 100%.

EXAMPLES 11-15:

In general accordance with the procedures of the preceding Example 10, several reaction mixtures are prepared and the reactants reacted. The composition of these reaction mixtures and the reaction conditions are summarized at Table II, runs 13-21, as are the results therefrom. While none of the reaction times exceed 40 minutes, conversions are as high as about 54% and epoxide selectivities average above 90%. This is all achieved using dilute aqueous $H_2O_2$ solutions.

COMPARATIVE EXAMPLE 2

In accordance with the procedures of Example 3, a 150 ml stainless steel bomb is charged with 20.659 g, 1,2-dichloroethane, 2.326 g 30% $H_2O_2$ in water (20.5 mmole), 0.0801 g phenylarsonic acid (0.4 mmole) and 1.0560 g benzene as an internal standard. The bomb is heated to 73°C in a water bath and pressurized to about 145 psig with propylene to dissolve it in the reaction medium in an amount in excess of the stoichiometric amount. Propylene

is added to maintain pressure and the bomb is shaken throughout. The results are summarized at Table III, run 1. After 120 minutes, a G.C. analysis shows no detectable amount of either propylene oxide or propylene glycol.

COMPARATIVE EXAMPLE 3

In general accordance with the procedures of Example 1, a 100 ml. three-neck flask equipped with a reflux condenser, thermometer, and magnetic stirrer is charged with 4.9332 g of cyclohexene 2.2890 g, 30% $H_2O_2$ in water (20.2 mmole), 20.85 g of 1,2-dichloroethane, and 0.0803 g of phenylarsonic acid (0.4 mmole). The reaction is stirred and heated at 73°C. After 1 hour, a G.C. analysis indicates the formation 0.206 g (2.1 mmole) of cyclohexene oxide and 0.088 g (0.8 mmole) of cyclohexandiol, i.e., a conversion to organics of 14.2%, and selectivity to cyclohexene oxide of 73.5%. After 3 hours, a G.C. analysis indicates the formation of 0.112 g (1.1 mmole) of cyclohexene oxide and 0.452 g (3.9 mmole) of cyclohexandiol (i.e., a conversion to organics of 24.9%, and selectivity to epoxide of 22.7%. The results are summarized at Table III, runs 23-24.

COMPARATIVE EXAMPLE 4

This example illustrates the effect of omitting Co-catalyst I from the reaction mixture when employing hexafluoroisopropanol as Co-catalyst II.

Into a 125 cc, 316 stainless steel Parr autoclave fitted with a thermocouple, propylene feed and vent lines is charged at ambient temperature olefin catalyst and oxidant to form a reaction mixture. The contents of the autoclave are then heated to reaction temperature while they are stirred with a stirring magnet. At the conclusion of the reaction, the reactor is cooled to ambient temperature, excess olefin is vented, and the contents analyzed by gas chromotography and iodometric titration. The reaction conditions and amounts of the components in the reaction mixture are summarized at

Table III, run 25, as are the results of the analysis.

COMPARATIVE EXAMPLE 5

This example is intended to illustrate the effect of using hexafluoroacetone solvent in conjunction with Co-catalyst I.

Accordingly, a 300 ml 316 stainless steel autoclave is charged with 0.6442 g of phenylarsonic acid, 4.93 g of 30% aqueous hydrogen peroxide solution and 50.05 g of hexafluoroacetone sesquihydrate. The mixture is heated to 77°C; then 126 psig of propylene (50.4g) is added with stirring. As the reaction proceeds, additional propylene was added to maintain pressure. After 15 minutes the autoclave was sampled and a G.C. analysis indicated the formation of 1.881 g of propylene glycol, and 0.0142 g of propylene oxide. After 30 minutes an analysis indicated the formation of 2.824 g propylene glycol and 0.0178 g of propylene oxide. The results are summarized at Table III runs 26 and 27.

**TABLE I**

**Epoxidation of Propylene Using Hydrogen Peroxide**

| Run No. | Ex. No. | Co-Catalyst I Phenyl-arsonic Acid (g) | Aqueous 30% $H_2O_2$ (g) | Co-Catalyst II (g) | Propylene (psig) | Reaction Temp. (°C) | Reaction Time (min.) | PRODUCTS (g) PO | PG | DPG | Conversion (%) To Organics | Total | Selectivities (%) PO | PG | DPG |
|---|---|---|---|---|---|---|---|---|---|---|---|---|---|---|---|
| 1 | 4 | 0.0804 | 2.3104 | HFI (24.715) $C_6H_6$(0.9675) | 145 | 71 | 167 | 1.495 | (c) | ND | 82 | ND | 100 | 0 | 0 |
| 2 | 5 | 0.0804 | 2.3158 | HFI (25.187) $C_6H_6$(1.0212) | 143 | 75 | 61 | 0.919 | (c) | ND | 78 | ND | 100 | 0 | 0 |
| 3 | 6 | 0.0800 | 2.4950 | HFI (25.50) $C_6H_6$(1.0416) | 126 | 70 | 60 | 1.144 | 0.062 | ND | 93.2 | 92.8 | 96.0 | 4.0 | 0 |
| 4 | 7 | 0.6442 | 4.91 | HFI (50.01) | 131 | 72 | 6 | 0.729 | 0.023 | 0.0 | 29.7 | ND | 97.0 | 2.4 | 0 |
| 5 | 7 | " | " | " | " | " | 16 | 1.419 | 0.094 | 0.048 | 60.1 | ND | 93.9 | 4.7 | 1.4 |
| 6 | 7 | " | " | " | " | " | 30 | 1.528 | 0.186 | 0.129 | 68.2 | 98.7 | 88.5 | 8.2 | 3.3 |
| 7 | 8 | 0.164 | 4.82 | HFI (50.44) | 139 | 72 | 5 | 0.494 | 0.014 | 0.013 | 20.7 | ND | 96.8 | 2.1 | 1.1 |
| 8 | 8 | " | " | " | " | " | 15 | 1.114 | 0.062 | 0.0 | 47.1 | ND | 95.9 | 4.1 | 0 |
| 9 | 8 | " | " | " | " | " | 30 | 1.418 | 0.151 | 0.030 | 62.6 | 84.3 | 91.7 | 7.5 | 0.8 |
| 10 | 9 | 0.6447 | 4.90 | *HFI (48.07) | 139 | 70 | 5 | 0.822 | 0.017 | 0.018 | 33.6 | ND | 97.5 | 1.5 | 1.0 |
| 11 | 9 | " | " | " | " | " | 15 | 1.645 | 0.052 | 0.039 | 67.8 | ND | 96.7 | 2.3 | 1.0 |
| 12 | 9 | " | " | " | " | " | 30 | 1.880 | 0.086 | 0.077 | 78.9 | 97.0 | 95.0 | 3.3 | 1.7 |

HFI = 1,1,1,3,3,3-hexafluoropropanol-2, $C_6H_6$ = benzene as internal standard
PO = propylene oxide, PG = 1,2-propylene glycol, DPG = dipropylene glycol
ND = Not determined
* = 2.002g heptane as internal standard

**TABLE II**

**Epoxidation of Propylene Using Hydrogen Peroxide**

| Run No. | Ex. No. | Co-Catalyst I Phenyl-arsonic Acid | Aqueous 30% $H_2O_2$ (g) | Co-Catalyst II (g) | Propylene (psig) | Reaction Temp. (°C) | Reaction Time (min.) | PRODUCTS(g) PO | PG | DPG | Conversion (%) To Organics | Total | Selectivities (%) PO | PG |
|---|---|---|---|---|---|---|---|---|---|---|---|---|---|---|
| 13 | 11 | 0.6423 | 11.44 | HFI (103.29) | 100 | 70 | 20 | 1.763 | 0.176 | ND | 32.4 | 34.8 | 92.9 | 7.1 |
| 14 | 11 | " | " | " " | " | " | 40 | 2.282 | 0.694 | " | 48.0 | 59.3 | 94.4 | 5.6 |
| 15 | 12 | 0.1611 | 5.09 | HFI (49.53) | 126 | 71 | 5 | 0.427 | 0.028 | " | 17.2 | ND | 95.2 | 4.8 |
| 16 | 12 | " | " | " " | " | " | 30 | 0.953 | 0.292 | " | 45.1 | 35.8 | 81.0 | 19.0 |
| 17 | 13 | 0.1610 | 5.41 | HFI (49.96) | 130 | 72 | 5 | 0.544 | 0.0 | " | 19.6 | 15.5 | 100 | 0 |
| 18 | 13 | " | " | " " | " | " | 30 | 1.124 | 0.288 | " | 48.5 | 57.6 | 83.6 | 16.4 |
| 19 | 14 | 0.1613 | 4.95 | HFI (49.69) | 133 | 70 | 30 | 1.064 | 0.253 | " | 49.5 | 79.2 | 84.6 | 15.4 |
| 20 | 15 | 0.6442 | 4.99 | HFI (51.91) | 535 | 74 | 5 | 0.547 | 0.016 | 0 | 21.4 | 48.5 | 97.8 | 2.2 |
| 21 | 15 | " | " | " " | " | " | 30 | 1.304 | 0.110 | 0 | 56.3 | 80.0 | 94.0 | 6.0 |

HFI = Hexafluoro isopropanol
PO = propylene oxide; PG = 1,2-propylene glycol; DPG = dipropylene glycol
ND = Not determined

TABLE III

| Run No. | Comp. Ex.No. | Co-Catalyst I Wt(g) | Co-Catalyst I Type | Aqueous $H_2O_2$ Sol. Wt (g) | Aqueous $H_2O_2$ Sol. $H_2O_2$ Conc. (0/0 %) | Co-Catalyst II Type | Co-Catalyst II Wt(g) | Olefin Type | Olefin psig | Reaction Temp(°C) | Reaction Time (min.) | Conversion (%) To Organics | Conversion (%) Total | Selectivities (%) Epoxide | Selectivities (%) Glycol |
|---|---|---|---|---|---|---|---|---|---|---|---|---|---|---|---|
| 22 | 2 | 0.0801 | PAA | 2.326 | 30 | DCE<br>B | 20.659<br>1.0560 | P | 145 | 73 | 120 | ND | ND | 0 | 0 |
| 23 | 3 | 0.0803 | PAA | 2.289 | 30 | DCE | 20.85 | $C_yH$ | 0 | 73 | 60 | 14.2 | ND | 73.5 | 26.5 |
| 24 | 3 | " | " | " | " | " | " | " | " | " | 180 | 24.9 | ND | 22.7 | 77.3 |
| 25 | 4 | None | None | 4.11 | 40 | HFIP | 50.56 | P | 100-150 | 70 | 60 | 10 | 12 | 73.3 | ND |
| 26 | 5 | 0.6442 | PAA | 4.93 | 30 | HFAS | 50.05 | P | 126 | 77 | 15 | 45.9 | 85.0 | 0.9 | 99.1 |
| 27 | 5 | " | " | " | " | HFAS | " | " | " | 77 | 30 | 70.7 | 98.9 | 0.3 | 99.7 |

PAA = Phenylarsonic Acid
P = Propylene
B = Benzene as internal standard
$C_yH$ = Cyclohexene
ND = Not determined
DCE = 1,2-dichloroethane
HFIP = Hexafluoro isopropanol
HFAS = Hexafluoroacetone sesquihydrate

DISCUSSION OF RESULTS

Referring to Tables I and II, it can be seen that in less than 3 hours reaction time conversions between 80 and 90% associated with selectivities of from 96 to 100% can be achieved. It is to be noted that these results are achieved using dilute aqueous solutions of $H_2O_2$. These results are substantially better for such short reaction times than those reported in U.S. Patent No. 4,024,165 which employs a tungsten catalyst, a nitrogen containing compound and hexafluoroisopropanol. Compare, for example, the results of the present invention with Example 31 of this patent wherein no reaction is reported after 3 hours.

Referring to Table III, run 22 illustrates the effect of substituting 1,2-dichloroethane (a non-oxygenated chlorinated solvent) for hexafluoroisopropanol, when employing propylene as the olefin, namely, no reaction takes place.

Runs 23 and 24 illustrate a similar effect as shown in run 22, when employing 1,2-dichloroethane instead of hexafluoroisopropanol, and cyclohexene as the olefin, namely, the yield of epoxide drops from 22.2% in Example 1 to 10.4% in run 23 and to 5.6% in run 24.

Run 25, which omits Co-catalyst I, results in a substantial drop in conversion (e.g., to 12%) and selectivity (e.g. to 73%). This is believed to illustrate that the presence of both Co-catalyst I and II is critical to obtain the results of the present invention.

Run 26, substitutes a fluorinated ketone sesquihydrate for hexafluoroisopropanol. The fluorinated ketone sesquihydrate results in the production of more glycol than epoxide. It is for this reason that hydrated ketones have been excluded from the claims herein.

EXAMPLE 16

The procedure of Example 10 is repeated using 0.8420 g of 4-hydroxy-3-nitrophenylarsonic acid, 48.67 g of hexafluoroisopropanol, 4.95 g of 30% $H_2O_2$ in water,

and 53.5 g of propylene. After 60 minutes at 76°C, there is obtained 0.875 g of propylene oxide, i.e., a conversion to organics of 40.5%, total conversion of 86.4%, and a selectivity to propylene oxide of 85.3%.

The principles, preferred embodiments, and modes of operation of the present invention have been described in the foregoing specification. The invention which is intended to be protected herein, however, is not to be construed as limited to the particular forms disclosed, since these are to be regarded as illustrative rather than restrictive. Variations and changes may be made by those skilled in the art without departing from the spirit of the invention.

CLAIMS

1. A process for epoxidizing at least one olefinic compound having at least one ethylenic unsaturation which comprises admixing said olefinic compound with hydrogen peroxide in the presence of a catalyst composition comprising at least one Co-Catalyst I and at least one Co-Catalyst II, in a manner and under conditions sufficient to oxidize at least one of said ethylenically unsaturated groups to its corresponding epoxide group, and wherein in said catalyst composition.

(i) said Co-catalyst I comprises at least one Group V element containing compound which is capable of catalyzing the oxidation reaction between hydrogen peroxide and the ethylenic unsaturation, said Group V element being selected from As, P, Sb, Bi, and mixtures thereof; and

(ii) said Co-catalyst II is at least one fluorinated oxygenated organic compound represented by the structural formulae selected from the group consisting of:

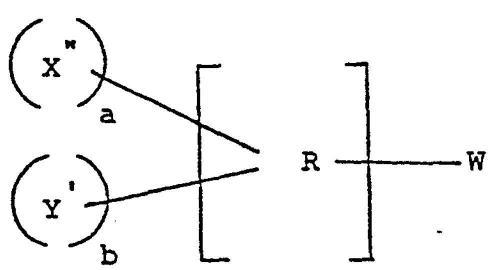

(I)

wherein: W is an oxygenated group selected from the group consisting of -OH, $-SO_3R_7$, $-COOR_8$, $-C-O-R_9$, $R_7$, $R_8$, and $R_9$, being independently selected from the group consisting of a halogen substituted or unsubstituted alkyl hydrocarbyl group of from about 1 to about 10 carbons, said oxygenated functional group being attached to an aliphatic carbon of R; R is selected from the hydrocarbyl group consisting of alkyl of from about 1 to about 10 carbons, aralkyl wherein the aryl portion thereof has from about 6 to about 10 carbons, and the alkyl portion thereof is as described above in connection with R, cycloalkyl of from about 4 to about 12 carbons; $X''$ is independently selected from the group

consisting of alkyl as described in connection with R above, hydrogen and halogen; $Y'$ is attached to the aliphatic portion of said R hydrocarbyl group and is independently selected from the group consisting of hydrogen, halogen, hydroxy, $-SO_3R_{11}$, $-COOR_{12}$, and $-C-O-R_{13}$, $R_{11}$, $R_{12}$, and $R_{13}$ being as defined in connection with $R_7$, $R_8$, and $R_9$ respectively, with the proviso that when $Y'$ is hydroxy its position of substitution on said R hydrocarbyl group is such that no aliphatic carbon of R contains more than one hydroxy group; the letter (a) represents a number of at least 1; at least one $X''$ is fluorine; the letter (b) represents a number such that the sum of a + b is a number equal to the total number of available carbon sites on the unsubstituted hydrocarbyl R group; and $X''$, $Y'$ and W bear an inert relationship with respect to each other on the same compound;

$$CF_3-\left[CF_2-\underset{Q}{C}-R_{26}\right]_n-CF_3 \qquad \text{(II)}$$

wherein: Q is an oxygenated radical selected from the group consisting of -OH, $-SO_3R_7$, $-COOR_8$, $-C-O-R_9$, $R_7$, $R_8$, and $R_9$ being as defined in connection with structural formula I; $R_{26}$ is selected from the group consisting of hydrogen and halogen with the proviso that when Q is OH, $R_{26}$ is hydrogen; and n is a number which can vary from about 5 to about 1000; and

$$CF_3-\left[CF_2-CF-Q'\right]_n-CF_3 \qquad \text{(III)}$$

wherein: $Q'$ is selected from the group consisting of -O- and $-\overset{O}{\overset{\|}{C}}-O-$, and n is as defined in connection with structural formula II.

2. The process of Claim 1 wherein said Co-catalyst I is selected from at least one member of the group consisting of:

(a) at least one inorganic Group V element containing compound selected from the group consisting of oxides, oxy acids, alkali metal oxy acid salts, alkaline earth metal oxy acid salts, halides, oxyhalides, thiohalides, sulfides, oxysulfides and metalides; and

(b) at least one organic Group V element containing compound represented by structural formula selected from the group consisting of:

$$R_1 Z X' Y,$$
$$R_1 R_2 Z X',$$
$$R_1 R_2 R_3 Z,$$
$$R_1 Z_1 (O) X' Y,$$
$$R_1 R_2 Z_1 (O) X'$$
and
$$R_1 R_2 R_3 Z_1 (O)$$

wherein: $R_1$, $R_2$, and $R_3$, which may be the same or different are hydrocarbyl groups selected from: unsubstituted alkyl of from about 1 to about 20 carbons, unsubstituted aryl of from about 6 to about 14 carbons, unsubstituted alkoxy, aryloxy, alkoxyarl, aryloxyalkyl, aralkyl, alkylthio, arylthio, and alkaryl wherein the alky and aryl groups thereof are as described immediately above in connection with alkyl and aryl respectively, unsubstituted cycloalkyl of from about 4 to about 20 carbons, or any two of said $R_1$, $R_2$, and $R_3$ groups together can constitute a cyclic hydrocarbon group having from about 4 to about 20 carbons; substituted alkyl, aryl, alkoxy, aryloxy, alkoxyaryl, aryloxyalkyl, alkaryl, alkylthio, arylthio, aralkyl, and cycloalkyl, said substituted hydrocarbyl groups being as defined immediately above in connection with the unsubstituted hydrocarbyl groups and said substituents being selected from the group consisting of halogen, nitro, and mixtures thereof;

and wherein X' and Y, which may be the same or different, are selected from the group consisting of hydrogen, halogen, hydroxy, the hydrocarbyl groups of alkyl, aryl, alkaryl, aralkyl, and alkoxy said hydrocarbyl groups being defined in connection with $R_{1 to 3}$ above, mercapto, acyloxy of the structural formula

$$-O-\overset{O}{\overset{\|}{C}}-R_4$$

, alkylthio of the structural formula $-SR_5$, and thioacyloxy of the structural formula

$$-O-\overset{S}{\overset{\|}{C}}-R_6$$

, wherein $R_4$, $R_5$, and $R_6$ are independently alkyl of from about 1 to about 20 carbons; Z represents one of said Group V elements in the plus 3 oxidation state; and $Z_1$ represents one of said Group V elements in the plus 5 oxidation state.

3. The process of Claim 2 wherein Co-catalyst I is represented by the structural formula:

$$R_1\ Z_1\ (O)\ X'\ Y$$

wherein X' and Y are hydroxy, $R_1$ is a halogen substituted or unsubstituted hydrocarbyl group selected from alkyl, aryl, aralkyl, and alkoxyaryl as defined in connection with $R_1$, and $Z_1$ represents said Group V element in the plus 5 oxidation state.

4. The process of Claim 1 wherein Co-catalyst I is represented by the structural formula

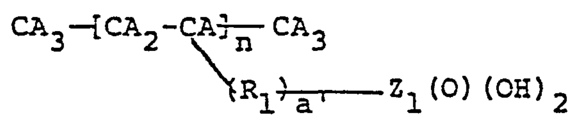

wherein $Z_1$ represents said Group V element in the plus 5 oxidation state, A is independently selected from hydrogen and halogen, $R_1$ is a hydrocarbyl group selected from alkyl of from about 1 to about 20 carbons, and aryl of from about 6 to about 14 carbons, a' is a number of 0 or 1, and n is a number which can vary from about 5 to about 1000.

5. The process of Claim 1 wherein the Group V element of Co-catalyst I is As; and Co-catalyst II is represented by structural formula I wherein W is -OH, X" is fluorine and Y' is selected from the group consisting of fluorine, hydrogen, and hydroxy.

6. The process of Claim 5 wherein Co-catalyst II is represented by structural formula I wherein W is hydroxy, X" is fluorine, Y' is hydrogen, and R is alkyl of from about 2 to about 8 carbons.

7. The process of Claim 5 wherein Co-catalyst II is represented by the structural formula:

$$R_{14}-CZ'_2-\overset{OH}{\underset{R_{15}}{C}}-R_{16} \qquad (IV)$$

wherein in structural formula IV: $R_{14}$, $R_{15}$, and $R_{16}$, which may be the same or different, are selected from the group consisting of hydrogen and a substituted or unsubstituted hydrocarbyl group selected from alkyl of from about 1 to about 10 carbons, aryl of from about 6 to about 10 carbons, aralkyl and alkaryl wherein the alkyl and aryl groups thereof are as defined immediately above, said hydrocarbyl group substituent being selected from hydroxy, halogen, and mixtures thereof and being substituted on the aliphatic portion of said $R_{14}$, $R_{15}$ and $R_{16}$ hydrocarbyl groups with the proviso that when the hydroxy and halogen substituents are present together on any of said hydrocarbyl groups, such respective substituents are located on different carbons; and wherein any two of said $R_{14}$, $R_{15}$, and $R_{16}$ groups together can constitute a substituted or unsubstituted cycloalkyl of from about 4 to about 12 carbons, said cycloalkyl substituents being as defined immediately above in connection with said $R_{14}$, $R_{15}$, and $R_{16}$ hydrocarbyl groups; and Z' is independently selected from the group consisting of halogen and hydrogen with the proviso that at least one Z' group is fluorine.

8. The process of Claim 7 wherein the halogen constituting any of: $Z'$ and the substituents of $R_{14}$, $R_{15}$, and $R_{16}$ is fluorine.

9. The process of Claim 5 wherein Co-catalyst II is represented by the structural formula selected from at least one member of the group consisting of:

$$CF_3-(CF_2)_{a'}CHOH-CF_3 \qquad (V)$$

$$\overline{\lfloor (CF_2)_{a'}CF_2-CF_2\ CHOH \rfloor} \qquad (VI)$$

and

$$CF_3-CHOH-(CF_2CHOH)_{a'}-(CF_2-CF_2)_{b'}-CF_3 \qquad (VII)$$

wherein in said structural formulae a' and b' are numbers which can independently vary from about 0 to about 10.

10. The process of Claim 1 wherein Co-catalyst II is represented by structural formula I and wherein in said structural formula I W is $-C-O-R_9$, $X''$ is fluorine, $Y'$ is hydrogen, and R is alkyl of from about 1 to about 8 carbons.

11. The process of Claim 1 wherein Co-catalyst II is represented by the structural formula selected from the group consisting of:

$$R_{23}-CG_2-O-CG_2-R_{24} \qquad (VIII)$$

and

$$CG_3-\left[OCG_2 - R_{25}\right]_n-CG_3 \qquad (IX)$$

wherein: $R_{23}$ and $R_{24}$ are independently selected from the group consisting of hydrogen, halogen, halogen substituted or unsubstituted alkyl of from about 1 to about 10 carbons and alkoxy wherein the alkyl portion is as described immediately above; $R_{23}$ and $R_{24}$ together can constitute halogen substituted or unsubstituted cycloalkyl of from about 4 to about 12 carbons; and G is independently selected from the

group consisting of hydrogen and halogen, with the proviso that at least one G group is fluorine; and wherein in structural formula (IX): $R_{25}$ is selected from the group consisting of halogen, hydrogen, and halogen substituted and unsubstituted alkyl of from about 1 to about 10 carbons, and n is a number of from about 1 to about 5.

12. The process of Claim 11 wherein the halogen independently constituting any of G, $R_{23}$, $R_{24}$, $R_{25}$, and halogen substituent of $R_{23}$, $R_{24}$, and $R_{25}$, is fluorine.

13. The process of Claim 1 wherein Co-catalyst I is selected from the group consisting of phenylarsonic acid, diphenylarsinic acid, 2-nitro-4 methylphenylarsonic acid, 4-methylphenylarsinic acid, n-propylarsonic acid, arsenic triethoxide, and mixtures thereof and Co-catalyst II is hexafluoroisopropanol.

14. The process of any one of Claims 1 to 13 wherein said epoxidation reaction is conducted by contacting in the liquid phase said olefin with a liquid reaction mixture comprising:

(a) hydrogen peroxide initially added to said reaction mixture as an aqueous solution, containing from about 20 to about 75%, by weight $H_2O_2$, based on the weight of said solution, in an amount of from about 0.5 to about 25%, by weight, based on the total weight of said reaction mixture exclusive of olefin and Co-catalyst I;

(b) Co-catalyst II in an amount of from about 10 to about 99.5%, by weight, based on the weight of said reaction mixture exclusive of the weight of olefin and Co-Catalyst I;

(c) inert organic solvent in an amount of from about 0 to about 90%, by weight, based on the weight of the reaction mixture exclusive of the weight of olefin and Co-Catalyst I; and

(d) Co-catalyst I in an amount sufficient to achieve from about 0.001 to about 2 g atoms of said Group V element in Co-catalyst I per liter of reaction mixture inclusive of components (a) through (c) recited above;

said reaction mixture being maintained at a temperature of from about 20 to about 150°C and the contacting of said olefin with said reaction mixture being conducted in a manner sufficient to achieve an initial molar ratio of olefin to $H_2O_2$ present in said reaction mixture of from about 0.8:1 to about 50:1.

15. The process of Claim 14 wherein: the hydrogen peroxide is initially added to the reaction mixture as an aqueous solution containing from about 20 to about 45% $H_2O_2$ and the $H_2O_2$ is present in said reaction mixture in an amount of from about 2 to about 20%, by weight, thereof, exclusive of the weight of olefin and Co-catalyst I; Co-catalyst II is present in said reaction mixture in an amount of from about 30 to about 90%, by weight thereof, exclusive of the weight of olefin and Co-catalyst I; the inert organic solvent is selected from the group consisting of benzene, toluene, xylene, pentane, hexane, heptane, isopropanol, acetone, propanoic acid, and ethyl acetate and is present in said reaction mixture in an amount of from about 0 to about 50%, by weight thereof, based on the weight of the reaction mixture exclusive of the weight of olefin and Co-catalyst I; and Co-catalyst I is present in said reaction mixture in an amount sufficient to achieve from about 0.005 to about 1 g atom of the Group V element of Co-catalyst I per liter of reaction mixture inclusive of components a through c, and wherein the initial molar ratio of olefin to $H_2O_2$ present in said reaction mixture is from about 1:1 to about 30:1.

16. The process of Claim 1 for epoxidizing an olefin selected from the group consisting of ethylene and propylene and mixtures thereof with $H_2O_2$ which comprises contacting, in the liquid phase, said olefin with a reaction mixture in a manner and under conditions sufficient to form a product selected from the group consisting of ethylene oxide, propylene oxide, and mixtures thereof, said reaction mixture comprising:

(a) hydrogen peroxide, initially added to the reaction mixture as an aqueous solution containing from about 20 to about 75% by weight $H_2O_2$, based on the weight of said solution, in an amount of from about 10 to about 15%, by weight based on the weight of the reaction mixture exclusive of the weight of olefin and Co-catalyst I;

(b) as Co-catalyst II hexafluoroisopropanol in an amount of from about 30 to about 90%, by weight, based on the weight of the reaction mixture exclusive of the weight of olefin and Co-catalyst I; and

(c) at least one Co-catalyst I selected from the group consisting of phenylarsonic acid, diphenylarsinic acid, 4-hydroxy-3 nitrophenyl arsonic acid, and mixtures thereof, in an amount sufficient to achieve from about 0.01 to about 1 g atom of arsenic per liter of reaction mixture inclusive of components (a) and (b).

0096130

European Patent Office

# EUROPEAN SEARCH REPORT

Application number

EP 82 30 3854

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,Y | US-A-3 993 673 (C.H. McMULLEN)<br>* Whole document *<br>--- | 1-16 | C 07 D 301/12<br>C 07 D 303/04 |
| D,Y | US-A-4 024 165 (T.M. SHRYNE)<br>* Whole document *<br>--- | 1-16 | |
| D,Y | GB-A-1 399 639 (SHELL)<br>* Whole document *<br>----- | 1-16 | |
| | | | TECHNICAL FIELDS SEARCHED (Int. Cl. 3) |
| | | | C 07 D 301/00<br>C 07 D 303/00 |

The present search report has been drawn up for all claims

| Place of search | Date of completion of the search | Examiner |
|---|---|---|
| THE HAGUE | 23-08-1983 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document